# EUROPÄISCHE PATENTSCHRIFT

(11) **EP 1 124 815 B1**
(45) Veröffentlichungstag und Bekanntmachung des Hinweises auf die Patenterteilung: **26.05.2004**
(21) Anmeldenummer: 99953803.6
(22) Anmeldetag: 13.10.1999
(51) Int. Cl.: C07D 311/30, A61K 7/42, A61K 31/35

(54) **VERFAHREN ZUR HERSTELLUNG VON LUTEOLIN UND LUTEOLIN-DERIVATEN**
METHOD FOR PRODUCING LUTEOLIN AND LUTEOLIN DERIVATIVES
PROCEDE DE PRODUCTION DE LUTEOLINE ET DE DERIVES DE LUTEOLINE

(30) Priorität: 30.10.1998 DE 19850283; 02.11.1998 DE 19850572
(43) Veröffentlichungstag der Anmeldung: 22.08.2001
(73) Patentinhaber: MERCK PATENT GmbH, 64293 Darmstadt (DE)
(72) Erfinder: BUCHHOLZ, Herwig, D-60599 Frankfurt (DE); ROSSKOPF, Ralf, D-64839 Münster (DE); LICHTENBERG, Alice, D-64285 Darmstadt (DE); KRAUS, Christine, D-01987 Schwarzheide (DE)
(86) Internationale Anmeldenummer: PCT/EP1999/007687
(87) Internationale Veröffentlichungsnummer: WO 2000/026206

(56) Entgegenhaltungen:
- EP-A- 0 633 022
- EP-A- 0 807 435
- FR-A- 2 699 818
- R. MOSQUERA ET. AL.: "Synthesis of O-(beta-hydroxyethyl) derivatives of Diosmetin." INDIAN JOURNAL OF CHEMISTRY, SECTION B, Bd. 35B, Nr. 1, Januar 1996 (1996-01), Seiten 19-22, XP000872742
- G. KITKEI ET. AL.: "Cyclodehydrogenation of 2'-Hydroxychalcones with Hypervalent Iodine Reagent. A New Synthesis of Flavones." LIEBIGS ANNALEN DER CHEMIE, Bd. 1995, 1995, Seiten 1711-5, XP002130763 in der Anmeldung erwähnt

## Beschreibung

Die Erfindung betrifft ein Verfahren zur Herstellung von Verbindungen der Formel I 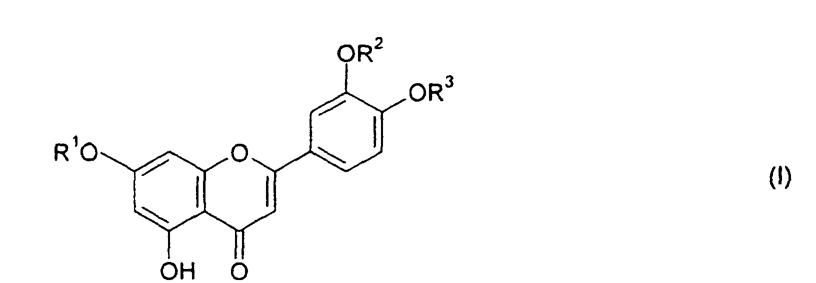 worin R¹ H oder (CH₂)ₘOH, R² H oder (CH₂)ₙOH, R³ H oder (CH₂)ₚOH, und m, n und p jeweils unabhängig voneinander 2 bis 8 bedeuten.

Die Verbindung der Formel I, in der R¹, R² und R³ H sind, ist Luteolin. Im Rahmen der vorliegenden Erfindung werden die Verbindungen der Formel I, in denen mindestens einer der Reste R¹, R² oder R³ eine andere Bedeutung als H besitzt, als Luteolin-Derivate bezeichnet.

Luteolin besitzt verschiedene vorteilhafte Eigenschaften. Luteolin ist ein hervorragendes Antioxidans und ein sehr guter Radikalfänger. Zudem inhibiert es sowohl enzymatische, nicht-enzymatische und CCl₄-induzierte Lipidperoxidationen. Luteolin besitzt einen günstigen Einfluß auf das Herz-Kreislaufsystem und kann der Entstehung von Artheriosklerose vorbeugen. Die antikanzerogene Wirkung von Luteolin zeigt sich unter anderem in der starken antiproliferativen Aktivität gegen unterschiedliche humane Tumor-Zell-Linien. Über entzündungshemmende, antivirale, antibakterielle und radioprotektive Eigenschaften von Luteolin wurde ebenfalls berichtet. Als Inhibitor des Enzyms Aldose Reductase kann Luteolin auch präventiv gegen die Entstehung diabetischer Katarakte wirken.

Die Luteolin-Derivate der Formel I besitzen ähnlich vorteilhafte Eigenschaften wie Luteolin selbst.

Bekannte Verfahren zur Herstellung der Verbindungen der Formel I haben den Nachteil, daß sie z.B. mehrere Synthesestufen umfassen und/oder unbefriedigende Ausbeuten an Produkt ergeben. Beispielsweise kann Luteolin bisher nur aus Pflanzen isoliert oder durch mehrstufige Synthesen hergestellt werden. Die Synthese aus geeigneten Chalconen und Hesperidin gelingt lediglich mit unbefriedigenden Ausbeuten [U. Achterrath-Tuckermann et al., Planta Med. 39 (1980) 38; D. Nagarathnam et al., J. Org. Chem. 56 (1991) 4884; Y.-H. Lu et al., Yao Hsueh Hsueh Pao 15 (1980) 477; G. Litkei et al., Liebigs Ann. 9 (1995) 1711; Y. Xing et al., Zhongguo Yiyao Gongye Zazhi 25 (1994) 484].

### Beschreibung des Standes der Technik:

EP 807 435 A2 betrifft die Verwendung von Luteolin, seiner Derivate und verwandter Verbindungen zur Herstellung eines Arzneimittels zur indirekten Hemmung der HMG-CoA-Reduktase.

EP 633 022 A2 beschreibt die Verwendung einer Flavonoid-Verbindung z.B. Luteolin zur Herstellung eines Knorpel schützenden Mittels, wobei dieses Mittel die Proteoglykan-Entleerung verhindert.

FR-A-2,699,818 beschreibt eine kosmetische oder pharmazeutische Verbindung, gekennzeichnet durch ein Antioxidantien-System, welches aus einem Gingko-Extrakt und mindestens einer Polyphenol-Verbindung (z.B. einem Flavonoid) zusammengesetzt ist.

Somit bestand die Aufgabe, ein Verfahren zur Herstellung der Verbindungen der Formel I zu entwickeln, welches die Nachteile bekannter Verfahren vermeidet oder zumindest vermindert, insbesondere eine einstufige Herstellung der Verbindungen der Formel I aus leicht zugänglichen Vorstufen ermöglicht und/oder die Herstellung der Verbindungen der Formel I in höherer Ausbeute ermöglicht.

Überraschend wurde nun gefunden, daß diese Aufgabe gelöst wird, wenn das Verfahren zur Herstellung der Verbindungen der Formel I 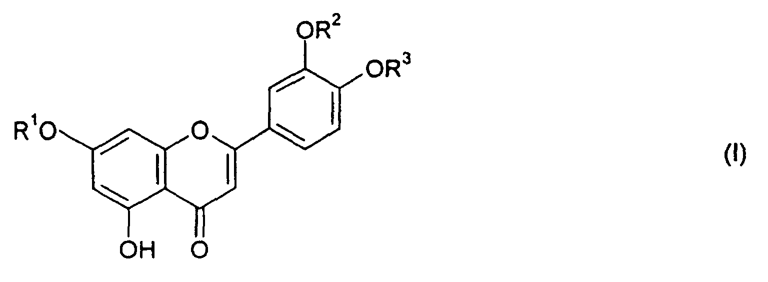 worin R¹ H oder (CH₂)ₘOH, R² H oder (CH₂)ₙOH, R³ H oder (CH₂)ₚOH, und m, n und p jeweils unabhängig voneinander 2 bis 8 bedeuten, so durchgeführt wird, daß Verbindungen der Formel II 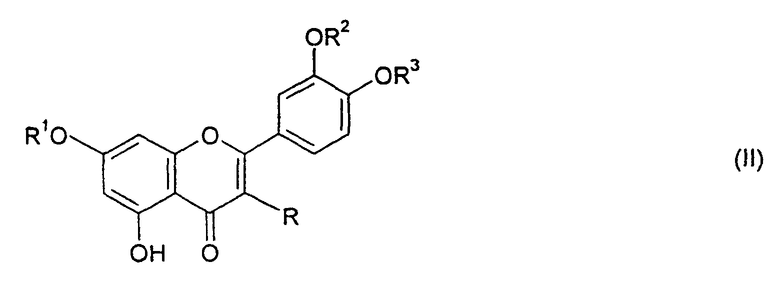 worin R¹, R² und R³ jeweils unabhängig voneinander die in Formel I angegebene Bedeutung besitzen, und 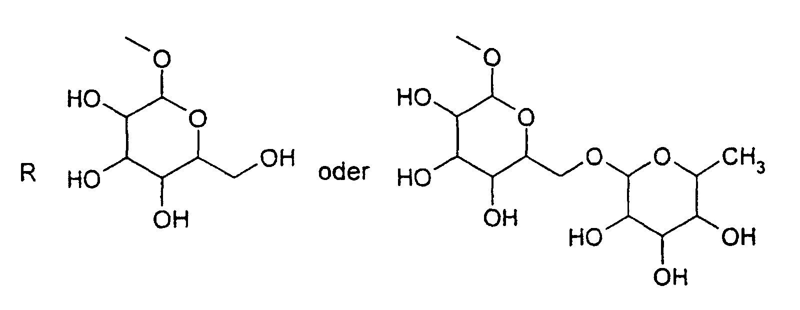 bedeutet, in wäßrigem alkalischen Milieu mit Natriumdithionit Na₂S₂O₄ reduziert werden.

Die Erfindung betrifft somit ein Verfahren zur Herstellung von Verbindungen der Formel I 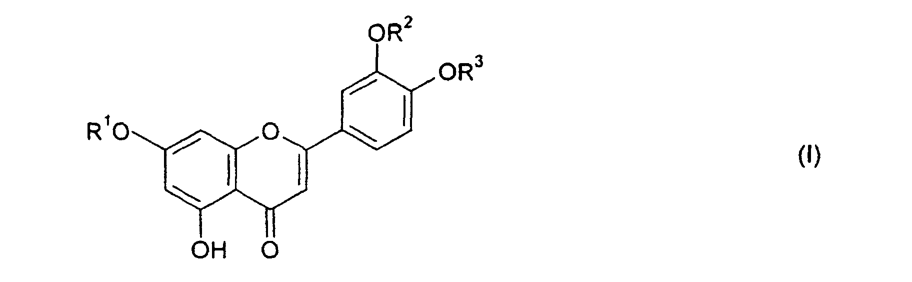 worin R¹ H oder (CH₂)ₘOH, R² H oder (CH₂)ₙOH, R³ H oder (CH₂)ₚOH, und m, n und p jeweils unabhängig voneinander 2 bis 8 bedeuten, dadurch gekennzeichnet, daß Verbindungen der Formel II 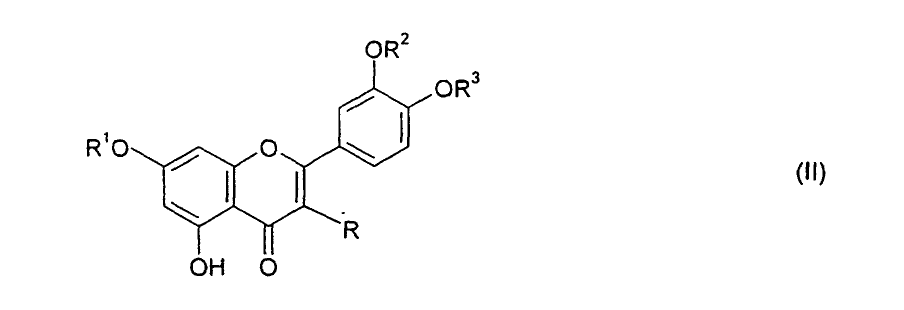 worin R¹, R² und R³ jeweils unabhängig voneinander die in Formel I angegebene Bedeutung besitzen, und 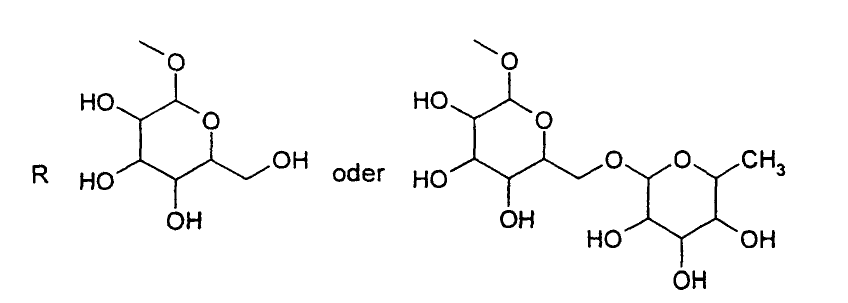 bedeutet, in wäßrigem alkalischen Milieu mit Natriumdithionit Na₂S₂O₄ reduziert werden.

Das erfindungsgemäße Verfahren zeichnet sich insbesondere dadurch aus, daß die Herstellung der Verbindungen der Formel I in einfacher Weise erfolgt und/oder in höheren Ausbeuten als es nach bisher bekannten Verfahren möglich war.

Die vorliegende Erfindung betrifft weiterhin Nahrungsmittel, die mit einer oder mehreren Verbindungen der Formel I angereichert worden sind sowie die Verwendung der Verbindungen der Formel I als Nahrungsergänzungsmittel.

Die Nahrungsmittel, die nach der vorliegenden Erfindung mit einer oder mehreren Verbindungen der Formel I angereichert werden können, umfassen alle Materialien, die für den Verzehr durch Tiere oder für den Verzehr durch Menschen geeignet sind, beispielsweise Vitamine und Provitamine davon, Fette, Mineralien oder Aminosäuren. Nahrungsmittel, die nach der vorliegenden Erfindung mit einer oder mehreren Verbindungen der Formel I angereichert werden können, sind beispielsweise auch Nahrungsmittel, die aus einer einzigen natürlichen Quelle stammen, wie z.B. Zucker, ungesüßter Saft, Nektar oder Püree von einer einzigen Pflanzenspezies, wie z.B. ungesüßter Apfelsaft (z.B. auch eine Mischung verschiedener Sorten Apfelsaft), Grapefruitsaft, Orangensaft, Apfelkompott, Aprikosennektar, Tomatensaft, Tomatensauce, Tomatenpüree, usw.. Weitere Beispiele für Nahrungsmittel, die nach der vorliegenden Erfindung mit einer oder mehreren Verbindungen der Formel I angereichert werden können, sind Korn oder Getreide einer einzigen Pflanzenspezies und Materialien, die aus derartigen Pflanzenspezies hergestellt werden, wie z.B. Getreidesirup, Roggenmehl, Weizenmehl oder Haferkleie. Auch Mischungen von derartigen Nahrungsmitteln sind geeignet, um nach der vorliegenden Erfindung mit einer oder mehreren Verbindungen der Formel I angereichert zu werden, beispielsweise Multivitaminpräparate, Mineralstoffmischungen oder gezuckerter Saft. Als weitere Beispiele für Nahrungsmittel, die nach der vorliegenden Erfindung mit einer oder mehreren Verbindungen der Formel I angereichert werden können, seien Nahrungsmittelzubereitungen, beispielsweise zubereitete Zerealien, Gebäck, Mischgetränke, speziell für Kinder zubereitete Nahrungsmittel wie Joghurt, Diätnahrungsmittel, kalorienarme Nahrungsmittel oder Tierfutter genannt.

Die Nahrungsmittel, die nach der vorliegenden Erfindung mit einer oder mehreren Verbindungen der Formel I angereichert werden können, umfassen somit alle genießbaren Kombinationen von Kohlehydraten, Lipiden, Proteinen, anorganischen Elementen, Spurenelementen, Vitaminen, Wasser und aktiven Metaboliten von Pflanzen und Tieren.

Die Nahrungsmittel, die nach der vorliegenden Erfindung mit einer oder mehreren Verbindungen der Formel I angereichert werden können sowie die Nahrungsergänzungsmittel, die eine oder mehrere Verbindungen der Formel I enthalten, werden vorzugsweise oral angewendet, z.B. in Form von Essen, Pillen, Tabletten, Kapseln, Pulver, Syrups, Lösungen oder Suspensionen.

Die mit einer oder mehreren Verbindungen der Formel I angereicherten erfindungsgemäßen Nahrungsmittel können mit Hilfe von Techniken hergestellt werden, die dem Fachmann wohl bekannt sind.

Weiterhin betrifft die Erfindung kosmetische oder pharmazeutische Formulierungen, welche eine oder mehrere Verbindungen der Formel I enthalten sowie die Verwendung einer oder mehrerer Verbindungen der Formel I in kosmetischen oder pharmazeutischen Formulierungen. Die kosmetischen Formulierungen, welche eine oder mehrere Verbindungen der Formel I enthalten sowie die Verwendung einer oder mehrerer Verbindungen der Formel I in kosmetischen Formulierungen ist bevorzugt.

Während vor etwa 30 Jahren Sonnenlicht aufgrund der Vitamin D-Synthese als heilend und unbedenklich angesehen wurde, hat sich in den letzten Jahren die Einstellung in dieser Beziehung nicht nur aus medizinischer Sicht erheblich geändert. Das Gefahrenpotential, welches sowohl natürliche als auch künstliche Bestrahlung mit Sonnenlicht in sich birgt, ist im Bewußtsein in den Vordergrund gerückt. Insbesondere ist auch eine Verhaltensänderung hervorgerufen worden durch das Wissen über den Einfluß von Sonnenlicht auf die Hautalterung und die Entstehung von Hautkrebs.

Bekanntlich reagiert die Haut empfindlich auf Sonnenstrahlen, welche einen gewöhnlichen Sonnenbrand oder ein Erythem, aber auch mehr oder weniger ausgeprägte Verbrennungen hervorrufen können.

Sonnenstrahlen haben aber auch andere negative Wirkungen: sie bewirken, daß die Haut ihre Elastizität verliert und sich Falten bilden und führen somit zu einer frühzeitigen Alterung. Manchmal kann man auch Dermatosen beobachten, und im extremen Fall kommt es zum Auftreten von Hautkrebs.

Es ist auch wünschenswert, Haare gegen photochemische Schäden zu schützen, um Veränderungen von Farbnuancen, ein Entfärben oder Schäden mechanischer Art zu verhindern.

Bekanntlich wird der gefährlichste Teil der Sonnenstrahlen von den ultravioletten Strahlen mit einer Wellenlänge von weniger als 400 nm gebildet. Bekannt ist auch, daß durch das Vorhandensein der Ozonschicht der Erdatmosphäre, die einen Teil der Sonnenstrahlung absorbiert, die untere Grenze der ultravioletten Strahlen, welche die Erdoberfläche erreichen, bei ca. 280 nm liegt.

Daher liegt das Hauptziel im Bereich Sonnenschutz eigentlich darin, einen guten Schutz gegen UVB- und UVA-Strahlung zu gewährleisten.

Die Verbindungen der Formel I können allein oder natürlich auch in Kombination mit weiteren Lichtschutzfiltern unterschiedlicher Substanzklassen, einzeln oder in Kombination, in der kosmetischen oder pharmazeutischen Zubereitung enthalten sein. Lichtschutzfilter anderer Substanzklassen sind beispielsweise organische oder anorganische UVAund UVB-Filter, IR- oder VIS-Filter. Insbesondere bevorzugt ist die Kombination mit organischen UV-Filtern oder deren Gemischen.

Gegenstand der Erfindung ist daher auch die Verwendung einer oder mehrerer Verbindungen der Formel I in kosmetischen Formulierungen wie Sonnenschutzmitteln, Hautcremes oder -gelen, Haargelen oder kosmetischen Stiften, insbesondere als UV-Filter.

Als weitere geeignete organische UV-Filter kommen alle dem Fachmann bekannten UVA- als auch UVB-Filter in Frage. Für beide UV-Bereiche gibt es viele aus der Fachliteratur bekannte und bewährte Substanzen, z.B.

Benzylidenkampferderivate wie
- 3-(4'-Methylbenzyliden)-dl-kampfer (z.B. Eusolex® 6300),
- 3-Benzylidenkampfer (z.B. Mexoryl® SD),
- Polymere von N-{(2 und 4)-[(2-oxoborn-3-yliden)methyl]benzyl}acrylamid (z.B. Mexoryl® SW),
- N,N,N-Trimethyl-4-(2-oxoborn-3-ylidenmethyl)anilinium methylsulfat (z.B. Mexoryl® SK) oder
- α-(2-Oxoborn-3-yliden)toluol-4-sulfonsäure (z.B. Mexoryl® SL),

Benzoyl- oder Dibenzoylmethane wie
- 1-(4-tert-Butylphenyl)-3-(4-methoxyphenyl)propan-1,3-dion (z.B. Eusolex® 9020) oder
- 4-Isopropyldibenzoylmethan (z.B. Eusolex® 8020),

Benzophenone wie
- 2-Hydroxy-4-methoxybenzophenon (z.B. Eusolex® 4360) oder
- 2-Hydroxy-4-methoxybenzophenon-5-sulfonsäure und ihr Natriumsalz (z.B. Uvinul® MS-40),

Methoxyzimtsäureester wie
- Methoxyzimtsäureoctylester (z.B. Eusolex® 2292),
- 4-Methoxyzimtsäureisopentylester, z.B. als Gemisch der Isomere (z.B. Neo Heliopan® E 1000),

Salicylatderivate wie
- 2-Ethylhexylsalicylat (z.B. Eusolex® OS),
- 4-Isopropylbenzylsalicylat (z.B. Megasol®) oder
- 3,3,5-Trimethylcyclohexylsalicylat (z.B. Eusolex® HMS),

4-Aminobenzoesäure und Derivate wie
- 4-Aminobenzoesäure,
- 4-(Dimethylamino)benzoesäure-2-ethylhexylester (z.B. Eusolex® 6007),
- ethoxylierter 4-Aminobenzoesäureethylester (z.B. Uvinul® P25),
und weitere Substanzen wie
- 2-Cyano-3,3-diphenylacrylsäure-2-ethylhexylester (z.B. Eusolex® OCR),
- 2-Phenylbenzimidazol-5-sulfonsäure sowie ihre Kalium-, Natrium- und Triethanolaminsalze (z.B. Eusolex® 232),
- 3,3'-(1,4-Phenylendimethylen)-bis-(7,7-dimethyl-2-oxobicyclo-[2.2.1]hept-1-ylmethansulfonsäure sowie ihre Salze (z.B. Mexoryl® SX) und
- 2,4,6-Trianilino-(p-carbo-2'-ethylhexyl-1'-oxi)-1,3,5-triazin ( z.B. Uvinul® T 150).

Diese organischen UV-Filter werden in der Regel in einer Menge von 0,5 bis 10 Gewichtsprozent, vorzugsweise 1 - 8 %, in kosmetische Formulierungen eingearbeitet.

Weitere geeignete organische UV-Filter sind z.B.
- 2-(2H-Benzotriazol-2-yl)-4-methyl-6-(2-methyl-3-(1,3,3,3-tetramethyl-1-(trimethylsilyloxy)disiloxanyl)propyl)phenol (z.B. Silatrizole®),
- 4,4'-[(6-[4-((1,1-Dimethylethyl)aminocarbonyl)phenylamino]-1,3,5-triazin-2,4-diyl)diimino]bis(benzoesäure-2-ethylhexylester) (z.B. Uvasorb® HEB),
- α-(Trimethylsilyl)-ω-[trimethylsilyl)oxy]poly[oxy(dimethyl [und ca. 6% methyl[2-[p-[2,2-bis(ethoxycarbonyl]vinyl]phenoxy]-1-methylenethyl] und ca. 1,5 % methyl[3-[p-[2,2-bis(ethoxycarbonyl)vinyl)phenoxy)propenyl) und 0,1 bis 0,4% (methylhydrogen]silylen]] (n ≈ 60) (z.B. Parsol® SLX),
- 2,2'-Methylen-bis-(6-(2H-benzotriazol-2-yl)-4-(1,1,3,3-tetramethylbutyl)phenol) (z.B. Tinosorb® M),
- 2,2'-(1,4-Phenylen)bis-(1H-benzimidazol-4,6-disulfonsäure, Mononatriumsalz) (z.B. Neo Heliopan® AP) und
- 2,4-bis-{[4-(2-Ethyl-hexyloxy)-2-hydroxyl]-phenyl}-6-(4-methoxyphenyl)-1,3,5-triazin (z.B. Tinosorb® S).

Diese organischen UV-Filter werden in der Regel in einer Menge von 0,5 bis 20 Gewichtsprozent, vorzugsweise 1 - 15 %, in kosmetische Formulierungen eingearbeitet.

Als anorganische UV-Filter sind solche aus der Gruppe der Titandioxide wie z.B. gecoatetes Titandioxid (z.B. Eusolex® T-2000), Zinkoxide (z.B. Sachtotec®), Eisenoxide oder auch Ceroxide denkbar. Diese anorganischen UV-Filter werden in der Regel in einer Menge von 0,5 bis 20 Gewichtsprozent, vorzugsweise 2 - 10 %, in kosmetische Formulierungen eingearbeitet.

In den Formulierungen, in denen Verbindungen der Formel I zusammen mit anderen UV-Filtern enthalten sind, wirken die Verbindungen der Formel I z.B. als Antioxidationsmittel und Radikalfänger. Zudem wird mit derartigen Formulierungen ein Breitband-UV-Schutz erreicht. Gegenstand der Erfindung ist somit auch die Verwendung von einer oder mehreren Verbindungen der Formel I in kosmetischen oder pharmazeutischen Formulierungen als UV-Filter, Antioxidans und/oder Radikalfänger.

Gegenstand der Erfindung ist ferner ein Verfahren zum Schutz der Haut und/oder natürlicher oder sensibilisierter Haare vor Sonnenstrahlen, wobei auf die Haut oder die Haare eine kosmetische Zubereitung, enthaltend eine oder mehrere Verbindungen der Formel I als Lichtschutzfilter, aufgetragen wird.

Gegebenenfalls können die erfindungsgemäßen Sonnenschutzmittel auch eine oder mehrere chemische Substanzen mit selbstbräunenden Eigenschaften enthalten.

Als chemische Substanzen mit selbstbräunenden Eigenschaften können alle dem Fachmann bekannten natürlichen und synthetischen Substanzen, welche zur Herstellung von kosmetischen Formulierungen geeignet sind, eingesetzt werden. Solche können sowohl pflanzliche Extrakte als auch synthetische Selbstbräuner, wie z.B. Dihydroxyaceton oder α-Ketole, sein.

Weiterhin können die erfindungsgemäßen Formulierungen auch zur vorbeugenden Behandlung von Entzündungen und Allergien der Haut sowie auch in bestimmten Fällen zur Verhütung bestimmter Krebsarten verwendet werden.

Die erfindungsgemäße Zubereitung wird als Mittel zum Schutz der menschlichen Epidermis oder der Haare oder auch der sensibilisierten Haare oder als Sonnenschutzmittel verwendet.

Mit "sensibilisierten Haaren" sind Haare gemeint, welche einer Dauerwellenbehandlung, einem Färbe- oder Entfärbeprozeß unterzogen worden sind.

Die erfindungsgemäße kosmetische Zubereitung wird zum Schutz menschlicher Epidermis gegen Sonneneinstrahlung verwendet. Dabei liegt sie in verschiedenen, für diesen Typ üblicherweise verwendeten Formen vor. So kann sie insbesondere als Lotion oder Emulsion, wie als Creme oder Milch (O/W, W/O), in Form ölig-alkoholischer, ölig-wäßriger oder wäßrig-alkoholischer Gele oder als feste Stifte vorliegen oder als Aerosol konfektioniert sein.

Die Formulierung kann kosmetische Adjuvanzien enthalten, welche in dieser Art von Zubereitungen üblicherweise verwendet werden, wie z.B. Verdickungsmittel, weichmachende Mittel, Befeuchtungsmittel, grenzflächenaktive Mittel, Emulgatoren, Konservierungsmittel, Mittel gegen Schaumbildung, Parfüms, Wachse, Lanolin, Treibmittel, Farbstoffe und/oder Pigmente, welche das Mittel selbst oder die Haut färben, und andere in der Kosmetik gewöhnlich verwendete Ingredienzien.

Man kann als Dispersions- bzw. Solubilisierungsmittel ein Öl, Wachs oder sonstigen Fettkörper, einen niedrigen Monoalkohol oder ein niedriges Polyol oder Mischungen davon verwenden. Zu den besonders bevorzugten Monoalkoholen oder Polyolen zählen Ethanol, i-Propanol, Propylenglycol, Glycerin und Sorbit.

Eine bevorzugte Ausführungsform der Erfindung ist eine Emulsion, welche als Schutzcreme oder -milch vorliegt und außer der oder den Verbindungen der Formel I als UV-Filter - und gegebenenfalls noch weiteren Lichtschutzfiltern - Fettalkohole, Fettsäuren, Fettsäureester, insbesondere Triglyceride von Fettsäuren, Lanolin, natürliche oder synthetische Öle oder Wachse und Emulgatoren in Anwesenheit von Wasser umfaßt.

Weitere bevorzugte Ausführungsformen stellen ölige Lotionen auf Basis von natürlichen oder synthetischen Ölen und Wachsen, Lanolin, Fettsäureestern, insbesondere Triglyceriden von Fettsäuren, oder öligalkoholische Lotionen auf Basis eines Niedrigalkohols, wie Ethanol, oder eines Glycols, wie Propylenglykol, und/oder eines Polyols, wie Glycerin, und Ölen, Wachsen und Fettsäureestern, wie Triglyceriden von Fettsäuren, dar.

Die erfindungsgemäße kosmetische Zubereitung kann auch als alkoholisches Gel vorliegen, welches einen oder mehrere Niedrigalkohole oder -polyole, wie Ethanol, Propylenglycol oder Glycerin, und ein Verdickungsmittel, wie Kieselerde umfaßt. Die ölig-alkoholischen Gele enthalten außerdem natürliches oder synthetisches Öl oder Wachs.

Die festen Stifte bestehen aus natürlichen oder synthetischen Wachsen und Ölen, Fettalkoholen, Fettsäuren, Fettsäureestem, Lanolin und anderen Fettkörpern.

Ist eine Zubereitung als Aerosol konfektioniert, verwendet man in der Regel die üblichen Treibmittel, wie Alkane, Fluoralkane und Chlorfluoralkane.

Soll das erfindungsgemäße Mittel natürliche oder sensibilisierte Haare vor Sonneneinstrahlung schützen, so kann es als Shampoo, Lotion, Gel oder Emulsion zum Ausspülen vorliegen, wobei die jeweilige Formulierung vor oder nach dem Schampoonieren, vor oder nach dem Färben oder Entfärben, vor oder nach der Dauerwelle aufgetragen wird; oder das Mittel liegt als Lotion oder Gel zum Frisieren und Behandeln, als Lotion oder Gel zum Bürsten oder Legen einer Wasserwelle, als Haarlack, Dauerwellmittel, Färbe- oder Entfärbemittel der Haare vor. Dieses Mittel kann außer der oder den Verbindungen der Formel I als organische UV-Filter - und gegebenenfalls weiteren Lichtschutzfiltern - verschiedene, in diesem Mitteltyp verwendete Adjuvanzien enthalten, wie grenzflächenaktive Mittel, Verdickungsmittel, Polymere, weichmachende Mittel, Konservierungsmittel, Schaumstabilisatoren, Elektrolyte, organische Lösungsmittel, Silikonderivate, Öle, Wachse, Antifettmittel, Farbstoffe und/oder Pigmente, die das Mittel selbst oder die Haare färben oder andere für die Haarpflege üblicherweise verwendete Ingredienzien.

Die erfindungsgemäßen kosmetischen Zubereitungen können mit Hilfe von Techniken hergestellt werden, die dem Fachmann wohl bekannt sind.

Unter den Verbindungen der Formel I sind die Verbindung, worin R¹, R² und R³ H bedeuten, und die Verbindungen, worin R¹ (CH₂)ₘOH, R² (CH₂)ₙOH und R³ (CH₂)ₚOH und m, n und p jeweils unabhängig voneinander 2 bis 8 bedeuten, bevorzugt. Diese Verbindungen der Formel I werden vor- und nachstehend auch als Verbindungen der Formel I* 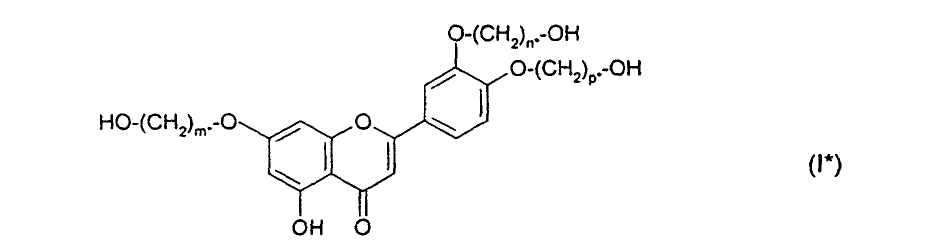 worin m*, n* und p* jeweils unabhängig voneinander 2 bis 8 bedeuten, bezeichnet. Besonders bevorzugt sind die Verbindungen der Formel I*.

Die vorliegende Erfindung betrifft auch diese Verbindungen der Formel I*.

In den Verbindungen der Formel I oder I* sind m, n und p oder m*, n* und p^{*} jeweils unabhängig voneinander 2, 3, 4, 5, 6, 7 oder 8. Vorzugsweise sind m, n und p oder m*, n* und p* jeweils unabhängig voneinander 2, 3 oder 4. Besonders bevorzugt sind m, n und p oder m*, n* und p* gleich, ganz außerordentlich bevorzugt bedeuten m, n und p oder m*, n* und p* 2.

Die Verbindungen der Formel I* besitzen eine bessere Wasserlöslichkeit als Luteolin selbst. Überraschenderweise wurde zudem gefunden, daß die Verbindungen der Formel I* farblos sind. Erfindungsgemäße Nahrungsmittel, die mit einer oder mehreren Verbindungen der Formel I* angereichert sind, sowie erfindungsgemäße kosmetische Formulierungen, die eine oder mehrere Verbindungen der Formel I* enthalten, sind besonders bevorzugt. Dementsprechend ist auch die Verwendung einer oder mehrerer Verbindungen der Formel I* als Nahrungsergänzungsmittel bzw. deren Verwendung in kosmetischen Formulierungen, z.B. als UV-Filter, besonders bevorzugt.

Die vorliegende Erfindung stellt ein vorteilhaftes Verfahren zur Herstellung der Verbindungen der Formel I durch Reduktion der Verbindungen der Formel II zur Verfügung. Dabei können entweder Rutin oder seine Derivate alleine, Isoquercetin oder seine Derivate alleine oder auch Mischungen davon, wie z.B. eine Mischung aus Rutin und Isoquercetin oder eine Mischung aus Rutinderivaten und Isoquercetinderivaten, als Edukte in die Reaktion eingesetzt werden.

Nach dem erfindungsgemäßen Verfahren werden die Verbindungen der Formel II in Wasser, vorzugsweise in siedendem Wasser, gelöst oder suspendiert und nach Einstellung eines alkalischen pH-Wertes, z.B. mit Alkali- oder Erdalkalihydroxiden oder -carbonaten wie Natronlauge oder Natriumcarbonat, Natriumdithionit Na₂S₂O₄ zugegeben. Anschließend wird das Reaktionsgemisch bis zum Ende der Reaktion gerührt, vorzugsweise unter Rückfluß.

Die Verbindungen der Formel II sind käuflich erwerbbar oder können nach Methoden, die dem Fachmann wohl bekannt und in der Literatur beschrieben sind (z.B. in Standard-Werken wie Houben-Weyl, Methoden der organischen Chemie, Georg-Thieme-Verlag, Stuttgart), gewonnen oder hergestellt werden.

Geeignete Reaktionstemperaturen für das erfindungsgemäße Verfahren sind Temperaturen zwischen 25 und 100 °C. Vorzugsweise wird das erfindungsgemäße Verfahren bei Reaktionstemperaturen von 50 bis 100 °C durchgeführt, insbesondere bei Reaktionstemperaturen von 80 bis 100 °C.

Geeignete pH-Werte für das erfindungsgemäße Verfahren sind pH-Werte zwischen 7,5 und 11. Vorzugsweise wird das erfindungsgemäße Verfahren bei pH-Werten von 8 bis 9 durchgeführt, insbesondere bei pH-Werten von 8,2 bis 8,7.

Geeignete Basen für das erfindungsgemäße Verfahren sind z.B. Alkalioder Erdalkalimetallhydroxide, -hydrogencarbonate oder -carbonate. Bevorzugte Basen sind ausgewählt aus NaOH, KOH, NaHCO₃, KHCO₃, Na₂CO₃, K₂CO₃. Besonders bevorzugt wird das erfindungsgemäße Verfahren unter Verwendung der Basen ausgewählt aus NaOH und Na₂CO₃ durchgeführt.

Geeignete Gewichtsverhältnisse der Verbindung der Formel II : Wasser für das erfindungsgemäße Verfahren sind Verhältnisse von 1 : 10 bis 1 : 200. Vorzugsweise wird das erfindungsgemäße Verfahren mit Gewichtsverhältnissen der Verbindung der Formel II : Wasser von 1 : 50 bis 1 : 150 durchgeführt, insbesondere mit Gewichtsverhältnissen von 1 : 80 bis 1 : 120.

Geeignete Gewichtsverhältnisse der Verbindung der Formel II : Natriumdithionit Na₂S₂O₄ für das erfindungsgemäße Verfahren sind Verhältnisse von 1 : 1 bis 1 : 100. Vorzugsweise wird das erfindungsgemäße Verfahren mit Gewichtsverhältnissen der Verbindung der Formel II : Natriumdithionit Na₂S₂O₄ von 1:5 bis 1 :40 durchgeführt, insbesondere mit Gewichtsverhältnissen von 1 : 8 bis 1 : 25.

Vorzugsweise wird das erfindungsgemäße Verfahren unter Atmosphärendruck durchgeführt.

Das Fortschreiten bzw. das Ende der Reaktion sowie die Analyse der Reaktionsprodukte kann z.B. mittels HPLC erfolgen, z.B. unter Verwendung von Standard-HPLC-Geräten und Säulen enthaltend reversed-phase-Materialien mit C₁₈-Alkyl-Belegung.

Alternativ kann das Fortschreiten bzw. das Ende der Reaktion auch mittels Dünnschichtchromatographie (DC) kontrolliert werden.

Nach beendeter Reaktion erfolgt die Isolierung des Reduktionsprodukts nach gängigen Methoden. Unter "üblicher Aufarbeitung" wird im Rahmen der vorliegenden Erfindung folgendes verstanden:

Das Reaktionsgemisch wird abgekühlt, beispielsweise auf eine Temperatur von 5 °C und anschließend durch Zugabe einer Säure wie z.B. Salzsäure neutralisiert. Vorzugsweise wird das Reaktionsgemisch nach der Zugabe der Säure weitergerührt, beispielsweise für 1 bis 12 h bei einer Temperatur von 0 oder 5 °C. Das hierbei auskristallisierende Rohprodukt wird vom restlichen Reaktionsgemisch abgetrennt, beispielsweise durch mechanische Methoden wie Absaugen oder Filtration. Die Reinheit des Rohprodukts ist üblicherweise > 96 %.

Zur weiteren Aufreinigung kann es z.B. mit vollentsalztem Wasser versetzt, einige Zeit unter Rückfluß gerührt und danach heiß filtriert werden, z.B. bei einer Temperatur von 85 °C. Die Reinheit des so erhaltenen Produkts ist üblicherweise > 99 %. Es können aber ohne weiteres auch Reinheiten > 99,5 % erzielt werden.

Abschließend wird der Feststoff getrocknet, z.B. für 12 h bei einem Druck von 200 mbar und einer Temperatur von 50 °C.

Auch ohne weitere Ausführungen wird davon ausgegangen, daß ein Fachmann die obige Beschreibung in weitestem Umfang nutzen kann. Die bevorzugten Ausführungsformen sind deswegen lediglich als beschreibende, keinesfalls als in irgendeiner Weise limitierende Offenbarung aufzufassen.

Die vollständige Offenbarung aller vor- und nachstehend aufgeführten Anmeldungen und Veröffentlichungen sind durch Bezugnahme in diese Anmeldung eingeführt.

Die folgenden Beispiele sollen die vorliegende Erfindung verdeutlichen. Sie sind jedoch keinesfalls als limitierend zu betrachten.

### Beispiele

Die Bezugsquellen für die verwendeten Substanzen sind wie folgt:

| | |
|---|---|
| Rutin | Merck KGaA, Artikel-Nr. 500017 |
| Trihydroxyethylrutin | Merck KGaA, Artikel-Nr. 501902 |

Die Reaktionskontrolle und die Analyse der Reaktionsprodukte erfolgt mittels HPLC.

| HPLC-Bedingungen bei Verwendung einer Standard-HPLC-Anlage: | | |
|---|---|---|
| Säule | LiChroSorb® RP18 (reversed phase-Material mit C₁₈-Alkyl-Belegung und einer Korngröße von 5 µm (Merck KGaA, Artikel-Nr. 151355)), | |
| Eluent | Gemisch aus Acetonitril und Wasser im Volumenverhältnis 20 : 80 (pH 2,6) | |
| Fluß | 1 ml/min, | |
| Wellenlänge | 260 nm, | |
| Temperatur | 30 °C, | |
| Probenvolumen | 10 µl, | |
| Probenvorbereitung | 5 mg der Probe in 3 ml Methanol lösen und mit dem Eluent auf 10 ml auffüllen, | |
| Retentionszeiten | Rutin | 7,3 min, |
| | Luteolin | 46,4 min, |
| | | |
| | Trihydroxyethylrutin | 10,6 min, |
| | Trihydroxyethylluteolin | 43,3 min. |

### Beispiel 1: Herstellung von Luteolin

60 g Rutin werden in 6 I vollentsalztem Wasser suspendiert und bei 100 °C zuerst 210 ml 32 %ige wäßrige Natronlauge und danach 600 g Natriumdithionit zugegeben. Anschließend wird weitere 12 h unter Rühren zum Rückfluß erhitzt, die Suspension auf 5 °C abgekühlt, langsam mit 195 ml rauchender Salzsäure neutralisiert und 1 h bei 0 °C gerührt. Nach üblicher Aufarbeitung werden 23,3 g Roh-Luteolin mit einer Reinheit von 96,5 % erhalten. Nach weiterer Aufreinigung und Trocknung werden 20,9 g Luteolin mit einer Reinheit von 99,6 % erhalten.

### Beispiel 2: Herstellung von Tri(hydroxyethyl)luteolin

5 g Tri(hydroxyethyl)rutin werden in 500 ml vollentsalztem Wasser gelöst und bei 100 °C zuerst 42,5 g Natriumcarbonat und danach 100 g Natriumdithionit zugegeben. Es wird eine weitere Stunde unter Rühren zum Rückfluß erhitzt, danach auf Raumtemperatur abgekühlt und weitere 72 h gerührt. Anschließend werden 65 ml rauchende Salzsäure bei 5 °C zugegeben und weitere 12 h gerührt. Nach üblicher Aufarbeitung werden 1,95 g Tri(hydroxyethyl)luteolin mit einer Reinheit von 98 % erhalten.

### Beispiel 3: Herstellung von Luteolin

3,5 g Isoquercetin werden bei 60 °C vorsichtig in 500 ml vollentsalztem Wasser suspendiert und in die resultierende gelbe Suspension 8,8 ml 32%ige wäßrige Natronlauge eingetragen. Hierbei entsteht eine dunkelrot gefärbte klare Lösung. Es werden 25 g Natriumdithionit bei 60 °C zugegeben und 12 h bei dieser Temperatur gerührt. Anschließend wird auf 5 °C abgekühlt und vorsichtig mit 37%iger Salzsäure neutralisiert, wobei die Lösung sofort eintrübt. Es wird 1 h bei 0 °C gerührt. Nach üblicher Aufarbeitung werden 2,05 g Luteolin mit einer Reinheit von 98,8% erhalten.

## Patentansprüche

1. Verfahren zur Herstellung von Verbindungen der Formel I 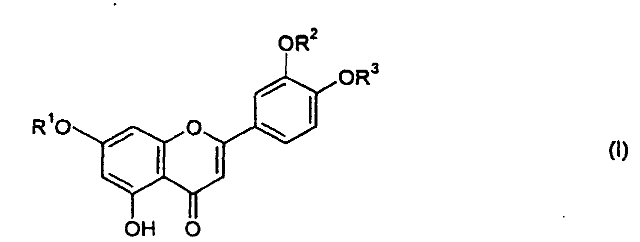 worin
R¹ H oder (CH₂)ₘOH,
R² H oder (CH₂)ₙOH,
R³ H oder (CH₂)ₚOH, und
m, n und p jeweils unabhängig voneinander 2 bis 8
bedeuten,
**dadurch gekennzeichnet, daß** Verbindungen der Formel II 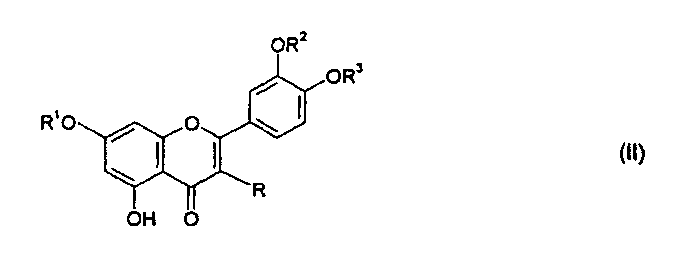 worin R¹, R² und R³ jeweils unabhängig voneinander die in Formel I angegebene Bedeutung besitzen, und 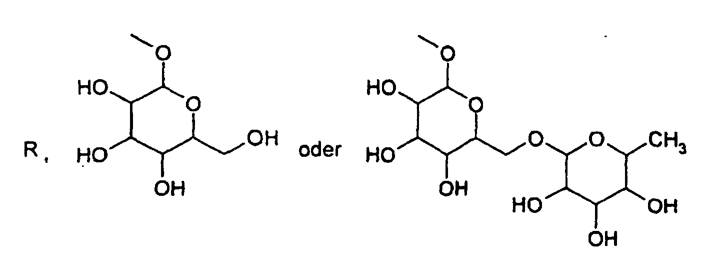 bedeutet, in wäßrigem alkalischen Milieu mit Natriumdithionit Na₂S₂O₄ reduziert werden.

2. Verfahren nach Anspruch 1, **dadurch gekennzeichnet, daß** eine Mischung aus Rutin und Isoquercetin oder eine Mischung aus Rutinderivaten und Isoquercetinderivaten als Edukt in die Reaktion eingesetzt werden.

3. Verfahren nach einem der Ansprüche 1 oder 2, **dadurch gekennzeichnet, daß** die Reaktion bei einer Reaktionstemperatur von 25 bis 100 °C durchgeführt wird.

4. Verfahren nach einem der Ansprüche 1 bis 3, **dadurch gekennzeichnet, daß** die Reaktion bei einem pH-Wert von 7,5 bis 11 durchgeführt wird.

5. Verfahren nach einem der Ansprüche 1 bis 4, **dadurch gekennzeichnet, daß** die Basen ausgewählt sind aus Alkali- oder Erdalkalimetallhydroxiden, -hydrogencarbonaten oder -carbonaten.

6. Verbindungen der Formel I* 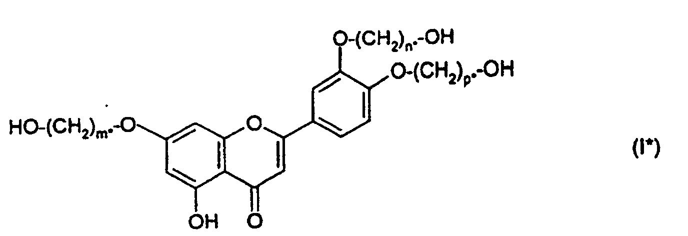 worin
m*, n* und p* jeweils unabhängig voneinander 2 bis 8
bedeuten.

## Claims

1. Process for the preparation of compounds of the formula I 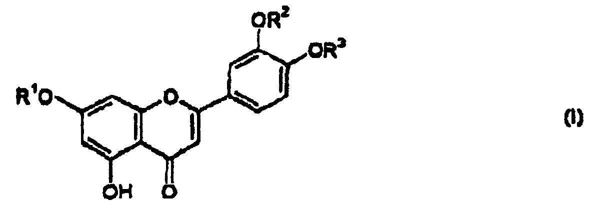 in which
R¹ is H or (CH₂)ₘOH,
R² is H or (CH₂)ₙOH,
R³ is H or (CH₂)ₚOH, and
m, n and p are each, independently of one another, from 2 to 8,
**characterised in that** compounds of the formula II 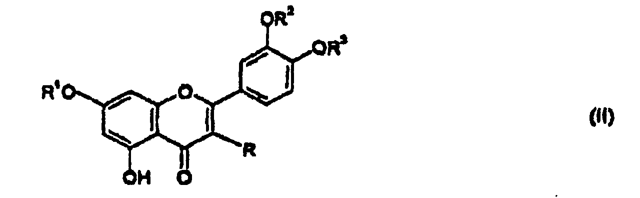 in which R¹, R² and R³ are each, independently of one another, as defined in the formula I, and 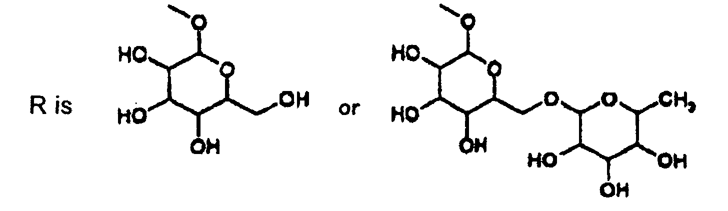 are reduced in aqueous alkaline medium using sodium dithionite Na₂S₂O₄.

2. Process according to Claim 1, **characterised in that** a mixture of rutin and isoquercetin or a mixture of rutin derivatives and isoquercetin derivatives is employed as starting material in the reaction.

3. Process according to one of Claims 1 and 2, **characterised in that** the reaction is carried out at a reaction temperature of from 25 to 100°C.

4. Process according to one of Claims 1 to 3, **characterised in that** the reaction is carried out at a pH of from 7.5 to 11.

5. Process according to one of Claims 1 to 4, **characterised in that** the bases are selected from alkali metal or alkaline earth metal hydroxides, hydrogencarbonates or carbonates.

6. Compounds of the formula I* 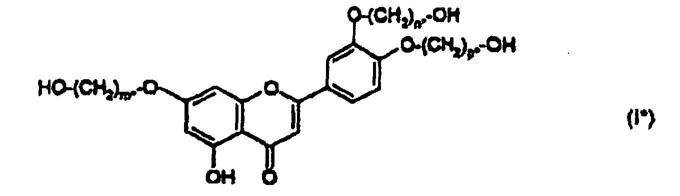 in which
m*, n* and p* are each, independently of one another, from 2 to 8.

## Revendications

1. Procédé pour la préparation de composés de la formule I 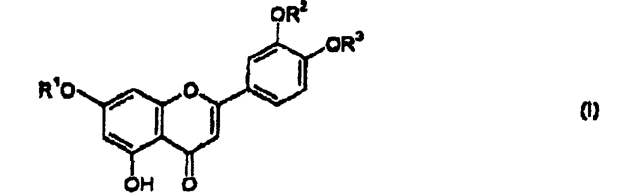 dans laquelle
R¹ représente H ou (CH₂)ₘOH,
R² représente H ou (CH₂)ₙOH,
R³ représente H ou (CH₂)ₚOH, et
m, n et p chacun, indépendamment l'un de l'autre, représente de 2 à 8,
**caractérisé en ce que** des composés de la formule II 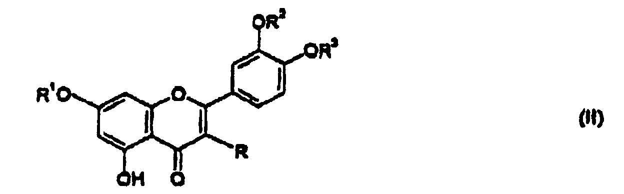 dans laquelle R¹, R² et R³ chacun, indépendamment l'un de l'autre, a la signification indiquée dans la formule I, et
R représente 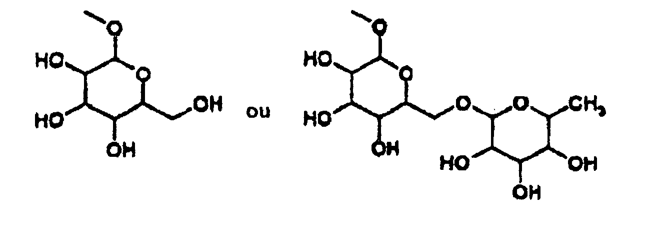 sont réduits dans un milieu alcalin aqueux en utilisant dithionite de sodium Na₂S₂O₄.

2. Procédé selon la revendication 1, **caractérisé en ce qu'**un mélange de rutine et d'isoquercétine ou un mélange de dérivés de rutine et de dérivés d'isoquercétine est employé en tant que matériau de départ dans la réaction.

3. Procédé selon l'une des revendications 1 et 2, **caractérisé en ce que** la réaction est mise en oeuvre à une température de réaction de 25 à 100°C.

4. Procédé selon l'une des revendications 1 à 3, **caractérisé en ce que** la réaction est mise en oeuvre à un pH de 7,5 à 11.

5. Procédé selon l'une des revendications 1 à 4, **caractérisé en ce que** les bases sont choisies parmi des hydroxydes de métal alcalin ou de métal alcalino-terreux, des carbonates hydrogénés ou des carbonates.

6. Composés de la formule I* 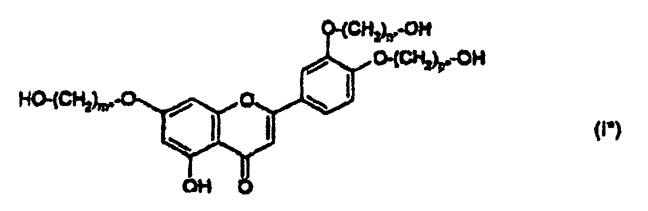 dans laquelle
m*, n* et p* chacun, indépendamment l'un de l'autre, représente de 2 à 8.
